# EUROPEAN PATENT SPECIFICATION

(11) **EP 2 969 001 B1**
(45) Date of publication and mention of the grant of the patent: **19.12.2018**
(21) Application number: 14778076.1
(22) Date of filing: 07.03.2014
(51) Int. Cl.: A61P 29/00, A61K 31/495, A61K 31/47, A61P 37/08

(54) **USE OF LEVOCETIRIZINE AND MONTELUKAST IN THE TREATMENT OF ANAPHYLAXIS**
VERWENDUNG VON LEVOCETIRIZIN UND MONTELUKAST BEI DER BEHANDLUNG VON ANAPHYLAXE
UTILISATION DE LEVOCETIRIZINE ET DE MONTELUKAST DANS LE TRAITEMENT DE L'ANAPHYLAXIE

(30) Priority: 13.03.2013 US 201361780452 P
(43) Date of publication of application: 20.01.2016
(73) Proprietor: IRR, Inc., Santa Barbara, CA 93103 (US)
(72) Inventor: MAY, Bruce, Chandler, Santa Barbara, California 93103 (US)
(74) Representative: Zacco Sweden AB
(86) International application number: PCT/US2014/021705
(87) International publication number: WO 2014/164281

(56) References cited:
- WO-A1-2008/100539
- WO-A1-2010/107404
- WO-A1-2011/003074
- WO-A1-2011/159821
- WO-A1-2013/012199
- US-A1- 2013 030 009
- TANG, ANGELA.: 'A Practical Guide to Anaphylaxis' AMERICA FAMILY PHYSICIAN vol. 68, no. 7, 01 October 2003, pages 1325 - 1332, XP055285208
- PERONI ET AL.: 'Combined Cetirizine-Montelukast Preventative Treatment for Food-Dependent Exercise-Induced Anaphylaxis' ANNALS OF ALLERGY, ASTHMA, & IMMUNOLOGY vol. 104, 01 March 2010, pages 272 - 273, XP026945045
- WANG XIUPING ET AL: "Montelukast Treatment of Acute Asthma Exacerbations in Children Aged 2 to 5 Years: A Randomized, Double-Blind, Placebo-Controlled Trial.", PEDIATRIC EMERGENCY CARE 06 JUN 2017, 6 June 2017 (2017-06-06), ISSN: 1535-1815

## Description

### CROSS-REFERENCE TO RELATED APPLICATIONS

This patent application claims the benefit of priority to U.S. Provisional Patent Application No. 61/780,452, filed March 13, 2013.

### BACKGROUND

The cornerstones of treatment for anaphylaxis are epinephrine and oxygen. Epinephrine is the drug of choice, with the appropriate dose given promptly on the onset of symptoms. The consensus of expert opinion supports the administration of care in the following order of importance: epinephrine, patient position (lying flat with the lower extremities elevated to preserve fluid in the circulation, prevent empty vena cava/empty ventricle syndrome, maintain the airway, and reduce the risk of aspiration), oxygen, intravenous fluids, nebulized therapy, vasopressors, antihistamines, and corticosteroids.

Currently, data establishing the efficacy of leukotriene modulators in the treatment of anaphylaxis or in its prevention has not been reported. Moreover, the only available route of administration is oral; thus, the onset of action of such agents in anaphylaxis may not be optimal.

Antihistamines, such as diphenhydramine (Benadryl®), are generally considered supportive therapy and do not replace epinephrine. Antihistamines are second line drugs that can be given after epinephrine administration since they may be useful for control of cutaneous and cardiovascular manifestations.

Debated in the scientific literature is whether the combination of an antihistamine plus leukotriene modulator for treatment in general offers an advantage over each alone. The contemporary literature is limited in this discrete area of medicine. In one chronic inflammatory disease state, chronic idiopathic urticaria, montelukast did not appear to offer an advantage over the second generation antihistamine desloratadine. Additionally, the FDA in April 2008 did not approve the combination of loratadine, a second generation antihistamine, plus montelukast for the treatment of allergic rhinitis and asthma.

Diphenhydramine, the prototype first generation antihistamine was developed by Swiss scientist, Daniel Bovet, between 1937-1944. Diphenhydramine has a large volume of distribution, 262 L/kg contrasted to an ideal molecule (<0.6 L/kg) which travels directly to the receptor to effect its response and is profoundly sedating. A Cochrane review concluded that there was no evidence from randomized controlled trials for the use of H-1 antagonists in treatment of anaphylaxis. Additionally, first generation antihistamines (FGAHs) were quoted as notorious for causing sedation and cognitive and psychomotor impairment; these side-effects may contribute to decreased awareness of anaphylaxis symptoms.

Other published literature agrees with the World Allergy Association in stating that while H-1 antagonists, both first and second generation antihistamines, may be useful in controlling cutaneous manifestations of anaphylaxis, there is no direct outcome data showing the effectiveness of antihistamines in anaphylaxis. Furthermore, epinephrine has far more clinical evidence to support its use over H-1 antihistamines in treatment of anaphylaxis. And while H-1 antihistamines are useful for relieving itching and urticaria, they do not relieve stridor, shortness of breath, wheezing, gastrointestinal symptoms, or shock.

IV montelukast (7 and 14 mg) has been tried as therapy for the treatment of asthmatics in an emergency room setting, and these studies showed the change in FEV1 (forced expiratory volume at one second) was significantly increased compared with placebo within 10 minutes of administration. However, the concept of using combined montelukast and levocetirizine has not been otherwise explored for acute care. PERONI et al. (Annals of Allergy, Asthma, & Immunology, 01 March 2010, Vol. 104, Pgs. 272-273) disclose that a combination of cetirizine and montelukast may be used as a preventive treatment for food-dependent exercise-induced anaphylaxis.

### SUMMARY

A combination of levocetirizine and montelukast in an effective amount for use in the treatment of anaphylaxis in a patient in need thereof is disclosed..

In another variation, the combination of levocetirizine and montelukast in an effective amount for use in treating a symptom of anaphylaxis in a patient in need thereof is disclosed..

In another variation, the combination of levocetirizine and montelukast in an effective amount for use in reducing the duration of an anaphylaxis in a patient in need thereof is disclosed..

The combination of levocetirizine and montelukast is administered at the onset of symptoms for any of the disclosed combinations for use.

The combination of levocetirizine and montelukast may be administered in a sequential manner for any of the disclosed combinations for use.

The combination of levocetirizine and montelukast may be administered in a substantially simultaneous manner for any of the disclosed combinations for use.

In some embodiments of the disclosed combinations for use, an additional active agent may be administered. The additional active agent may be a histamine H2 receptor antagonist. In one embodiment, the histamine H2 antagonist is ranitidine. In some embodiments, the histamine H2 antagonist is cimetidine.

In some embodiments of the disclosed combinations for use, the additional active agent may be a beta-2 agonist. In some embodiments, the additional active agent may be a glucocorticoid. In some embodiments, the additional active agent may be a H1-antihistamine. In a variation, the additional active agent may be oxygen. In another variation, the additional active agent may be saline.

In some embodiments of the disclosed combinations for use, a vasoactive agent may be administered. The vasoactive agent may be epinephrine. In some embodiments, the vasoactive agent is dopamine.

In some embodiments of the disclosed combinations for use, the combination may be administered to the patient by one or more of the routes consisting of enteral, intravenous, intraperitoneal, inhalation, intramuscular, subcutaneous and oral.

In some embodiments, the levocetirizine and montelukast may be administered by the same route.

Also disclosed in the present disclosure are methods, formulations and kits for treating anaphylaxis.

The methods and formulations include, but are not limited to, methods and formulations for delivering effective concentrations of levocetirizine and montelukast to a patient in need. The methods and formulations can comprise conventional and/or modified-release elements, providing for drug delivery to the patient.

According to the present disclosure, a combination of levocetirizine and montelukast, either as a single formulation or as separate formulations, may be administered as an emergency medication. For example, according to the present disclosure, a combination of levocetirizine and montelukast, either as a single formulation or as separate formulations, may be administered immediately at the onset of symptoms. According to the present disclosure, a combination of levocetirizine and montelukast, either as a single formulation or as separate formulations, may be administered substantially close to the onset of symptoms.

According to the present disclosure, the methods of treatment, formulations and kits may include e.g., a bilayer tablet, comprising levocetirizine and montelukast in separate layers, for daily administration, for example, to prevent recurrent or treat persistent symptoms, e.g., biphasic or protracted (refractory) reactions. Alternatively, each medication may be administered separately (one tablet of levocetirizine and one tablet of montelukast per day in the evening). According to the present disclosure, a combination of levocetirizine and montelukast, either as a single formulation or as separate formulations, may be administered for 1, 2, 3, 4, 5, 6, 7, 8, 9, 10, 11, 12, 13, 14, or 15 days for the treatment of anaphylaxis and associated conditions within the definition. The bilayer tablets or the separate tablets may be packaged in a blister pack supplied for a 7 to 15 day course of therapy, with instructions including indications, administration instructions and precautions. The bilayer tablets or the separate tablets may be packaged in a blister pack supplied for up to a 30 day course of therapy, with instructions including indications, administration instructions and precautions.

### BRIEF DESCRIPTION OF THE DRAWINGS

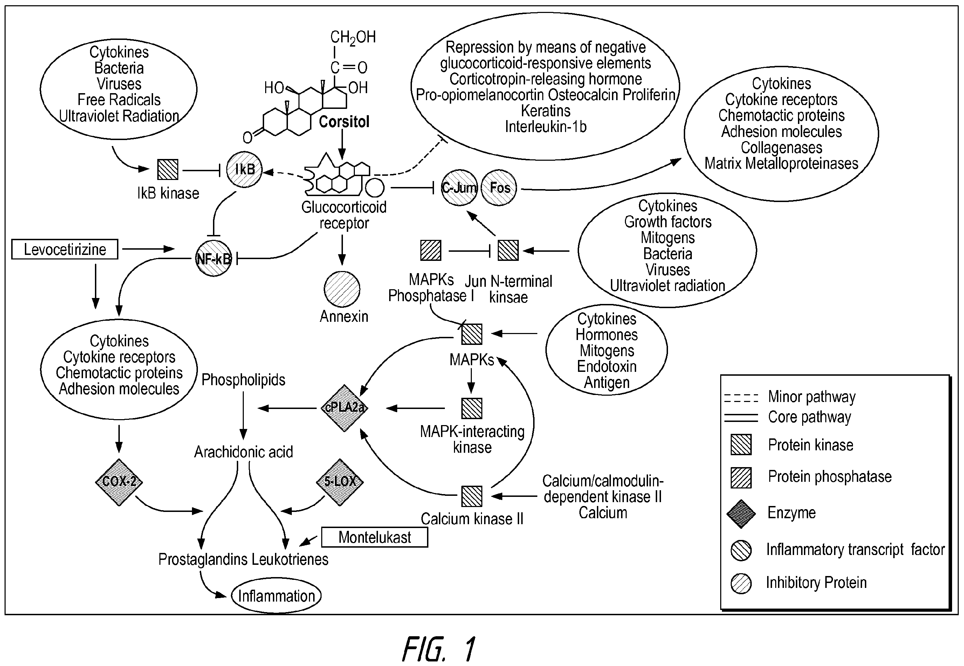

Figure 1 shows a diagram of the proposed anti-inflammatory mechanism of action of levocetirizine and montelukast utilizing a steroid model pathway. Corticosteroids switch off transcription of activated genes that encode pro-inflammatory proteins: they block or decrease the late phase allergic response.

### DETAILED DESCRIPTION

The present embodiments relate to the combination of levocetirizine and montelukast for use in the treatment of acute allergic reactions, such as anaphylaxis and anaphylactic-like reactions. Administration of levocetirizine and montelukast in combination exhibits synergistic effects and unexpectedly superior results in the treatment of such acute allergic reactions. Moreover, combinations of levocetirizine and montelukast can be used safely in conjunction with many existing treatment protocols.

Levocetirizine is an antihistamine and montelukast is a leukotriene receptor antagonist. As described herein, synergy between levocetirizine and montelukast shortens the course of the disease processes, thereby decreasing morbidity and mortality. This combined therapy also can improve quality of life from the amelioration of symptoms / side effects / disease process itself, and can decrease health-care costs. This synergistic effect can be observed in the use of a combination of levocetirizine and montelukast to treat non-lgE-mediated inflammation and combined non-lgE-mediated and IgE-mediated inflammation. Not wishing to be bound by a particular theory, the non-lgE-mediated response may be related, at least in part, to the fact that both levocetirizine and montelukast affect eosinophil migration, the leukocyte that is considered a hallmark of inflammation

Levocetirizine, a potent H1- antihistamine, acts primarily by down-regulating the H1 receptor on the surface of mast cells and basophils to block the IgE-mediated release of histamine which cause the cardinal symptoms of allergic rhinitis: sneezing, rhinorrhea, nasal congestion, itchy palate and itchy red and watery eyes. Levocetirizine offers a short time to peak plasma level, 0.9 hr., a short time to steady state level, 40 hours, a low volume of distribution, 0.4 L/kg, and an enhanced receptor affinity of 5x over first generation mepyramine in an acidic pH (many acute inflammatory disease states are associated with acidosis, a low physiologic pH). Levocetirizine has a 24 hour receptor occupancy of -75%, the highest of the commercially available antihistamines. Receptor occupancy of the second generation antihistamines appears to correlate with the pharmacodynamic activity in skin wheal and flare studies and with efficacy in allergen challenge chamber studies. Levocetirizine is approved in the US for the treatment of perennial allergic rhinitis and chronic idiopathic urticaria down to six months of age.

Levocetirizine has been objectively established as the most potent of the five modern generation antihistamines through histamine induced wheal and flare data. For example, levocetirizine at 5 mg per day is more effective than fexofenadine at its commonly prescribed dose of 180 mg per day in the United States. In Europe the adult dose is 120 mg per day. Levocetirizine has a lower volume of distribution, greater histamine receptor affinity in an inflamed state (low pH), and greater receptor occupancy at 24 hours at physiologic doses than fexofenadine. The corresponding values are shown in Table I.

**TABLE I**

| COMPARISON BETWEEN FEXOFENADINE AND LEVOCETIRIZINE | | |
|---|---|---|
| | Fexofenadine | Levocetirizine |
| Vd -L/kg | 5.6 L/kg | 0.4 L/kg |
| Receptor affinity in an acidic ph | increased 2x | increased 5x |
| Histamine receptor occupancy at 24 hours | ∼25% | ∼75% |
| Steady-state level | 3 days | 40 hours |

Levocetirizine decreases human rhinovirus titers in vitro by log-2. Not to be bound by a particular theory, the cellular mechanism of action is a proposed reduction of the activation of the intracellular protein complex NF-kB (nuclear factor kappa B) which is in turn responsible for the reduction of I-CAM-1. I-CAM-1, a transmembrane protein, is viewed as the portal of entry of human rhinovirus into the cell. Rhinovirus can be found in - 50% of cases of acute asthma and is responsible for 30-50% cases of the 'common cold.' A one-log reduction in viral titers has been independently determined to correlate with improved symptoms. In addition, levocetirizine has been shown to decrease eosinophil migration and decrease inflammatory mediators, IL-4, IL-6, and IL-8. IL-6, a signaling protein, regulates in part: fever, the body's response to trauma, and the acute (immediate) phase of the allergic reaction.

Montelukast, a leukotriene receptor antagonist, acts by binding with high affinity and selectivity to the CysLT1 receptor to inhibit the physiologic actions of the leukotriene LTD4. Leukotrienes are fatty signaling molecules whose effects include airway edema, smooth muscle contraction and altered cellular activity associated with the inflammatory process. Overproduction of leukotriene is a major cause of inflammation in asthma and allergic rhinitis. The cysteinyl leukotrienes (LTC4, LTD4, LDE4) are products of arachidonic acid metabolism. These leukotrienes are released from various cells including mast cells and eosinophils. They bind to receptors in the human airway and on other pro-inflammatory cells including eosinophils and certain myeloid stem cells. The cysteinyl leukotrienes have been correlated with the pathophysiology of asthma and allergic rhinitis.

Leukotriene D4 is the most potent of the cysteinyl leukotrienes in contracting airway smooth muscle. Leukotriene receptors, such as CysLTi, are found throughout the cells of the respiratory tree (including airway smooth muscle cells and airway macrophages) as well as on other pro-inflammatory cells in the body, particularly eosinophils and certain myeloid stem cells. Leukotrienes also function to promote the recruitment of eosinophils, dendritic cells and T cells. Eosinophil infiltration is considered by some authorities as a hallmark of inflammation.

Montelukast is FDA approved in the US for the treatment of perennial allergic rhinitis, asthma, seasonal allergic rhinitis, and exercised induced bronchospasm. Montelukast has been shown to be ineffective in improving asthma control or cold symptom scores caused by experimental rhinovirus infection. See Kloepfer KM, et al., Effects of montelukast in patients with asthma after experimental inoculation with human rhinovirus 16. Annals Allergy Asthma Immunology. 2011;106:252-257. Unlike levocetirizine, no decrease in viral shedding was observed in rhinovirus-infected individuals treated with montelukast and there was no significant difference in reported cold symptom scores compared to placebo-treated individuals. Analysis of secondary outcomes suggests that montelukast may protect against reductions in lung function and increases in sputum eosinophils caused by common cold infections. During the recovery phase the percentage of sputum eosinophils was elevated in the placebo group, while the montelukast group remained at baseline levels. Further, peak expiratory flow was not decreased in the montelukast-treated patients. Other studies have shown that montelukast treatment has no effect on the respiratory symptoms of patients with acute respiratory syncitial virus bronchiolitis. See Bisgaard, H., et al., Study of montelukast for the treatment of respiratory symptoms of post-respiratory syncitial virus bronchiolitis in children, Am. J. Respir. Crit. Care Med., 2008; 178:854-860; and Proesmans, M., et al., Montelukast does not prevent reactive airway disease in young children hospitalized for RSV bronchiolitis, Acta Paediatr. 2009; 98:1830-34. However, some studies indicate that treatment with montelukast reduced the number of days with worsened asthma symptoms and unscheduled doctor's visits in children with mild allergic asthma and resulted in a modest reduction of symptoms in children with recurrent wheezing when given at the first sign of upper respiratory tract illness. See Sears, M.R. and Johnston, N.W., Understanding the September asthma epidemic. J. Allergy Clin. Immunol. 2007; 120:526-29; Bacharier, L.B., et al., Episodic use of an inhaled corticosteroid or leukotriene receptor antagonist in preschool children with moderate-to-severe intermittent wheezing. J. Allergy Clin. Immunol. 2008; 122:1127-35.

Montelukast reaches a steady state level, like the second generation antihistamine, levocetirizine, in less than two days. Unlike other currently available leukotriene modulators, zileuton and zafirlukast, routine monitoring of liver function tests is not required. There are no drug interactions with warfarin, theophylline, digoxin, terfenadine, oral contraceptives, or prednisone.

The two molecules are safe, i.e., FDA approved in the United States for allergic disorders down to age six months. They can be given primarily or in conjunction with many of the existing therapeutic protocols for the treatment of inflammation, including but not limited to, influenza, acute asthma and the common cold. Both medications are pregnancy category B (Table II).

**TABLE II**

| PREGNANCY CATEGORY DEFINITIONS | | |
|---|---|---|
| Category | Definition | Explanation |
| A | Generally acceptable | Controlled studies in pregnant women show no evidence of fetal risk. |
| B | May be acceptable | Either animal studies show no risk but human studies not available or animal showed minor risks and human studies were done and showed no risk. |
| C | Use with caution if benefits outweigh risks | Animal studies show risk and human studies not available or neither animal nor human studies were done. |
| D | Use in life-threatening emergencies when no safer drug is available | Positive evidence of human fetal risk. |
| X | Do not use in pregnancy | Risks involved outweigh potential benefits. Safer alternatives exist. |

Existing treatment of inflammation focuses on the underlying condition and nature of the presentation. Commonly employed are a myriad of agents such as: diphenhydramine (Benadry10), oxygen, epinephrine, steroids, beta-agonists, non-steroidal anti-inflammatory agents (NSAIDS), antipyretics, antibiotics, antifungals, and antivirals. Paradoxically, the commonly employed NSAIDS actually increase the production of leukotrienes.

Steroids, which are widely used to treat inflammation, have significant short and long-term side-effects (Table III). With regard to treating inflammation associated with rhinosinusitis, nasal steroids have their limitations, particularly in the elderly and those patients on aspirin, clopidogrel or warfarin prescribed to reduce the risk of stroke and heart attack. Even in patients who do not take these traditional "blood thinners," the risk of spontaneous epistaxis from nasal steroid sprays is between 4-22%. The risk of epistaxis is medication dependent. Epistaxis is a significant consideration in many patients 55 or older.

**TABLE III**

| STEROID SIDE EFFECTS | |
|---|---|
| Short term | Long term |
| Increased propensity for opportunistic | Glaucoma |
| infection | Cataracts |
| Increased blood pressure | High-blood pressure |
| Mood changes | Heart disease |
| Increased blood sugar | |
| Increased intraocular pressure | Diabetes mellitus |
| Water retention | Obesity |
| Weight gain | Acid reflux/GERD |
| Increased risk for congestive heart failure | Osteoporosis |
| Flushing | Myopathy |
| Increased appetite | Increased propensity for opportunistic infection |
| Insomnia | Cushing syndrome |

The typical daily dosage for levocetirizine is 5mg for adults, and levocetirizine exhibits the following advantageous properties: i) Short time to reach peak plasma levels - 0.9 hr; ii) Short time to steady state level - 40 hrs; iii) Low volume of distribution (goes directly to the target receptor); iv) High receptor occupancy at 24 hours 75%; v) Increased receptor affinity in inflamed tissue (acidic pH; up to 5x that of first generation molecules); vi) Pregnancy category B; vii) FDA approved down to six months for other disease states, i.e., perennial allergic rhinitis and chronic idiopathic urticaria; viii) Anti-inflammatory properties; and ix) Anti-viral properties. Studies in humans have shown that doses of levocetirizine up to 30 mg/day can be safely administered.

Montelukast, a leukotriene receptor antagonist, acts concurrently to protect the respiratory tree as well as block mediators in the inflammatory cascade. The typical daily dosage of montelukast is 10 mg for adults, and montelukast exhibits the following advantageous properties: i) montelukast is a selective receptor antagonist, inhibiting the physiologic action of LTD4 at the CysLTi receptor; ii) montelukast binds with high affinity and selectivity to the CysLTi receptor without producing any agonist activity; iii) montelukast is rapidly absorbed; iv) montelukast reaches a peak plasma concentration in 3-4 hours; v) the oral bioavailability and Cₘₐₓ of montelukast are not affected by a standard meal; vi) montelukast has a linear pharmacokinetics to 50 mg; vii) doses as low as 5 mg in adults cause substantial blockage of LTD4-induced bronchoconstriction; viii) in a placebo controlled crossover study, montelukast inhibited early-phase bronchoconstriction due to antigen challenge by 75%; ix) montelukast is FDA approved down to six months of age; and x) montelukast has no drug interactions with warfarin, theophylline, digoxin, terfenadine, oral contraceptives, or prednisone. Montelukast has been administered at doses up to 200 mg/day to adult patients for 22 weeks and in short-term studies, and up to 900 mg/day to patients for approximately one week without clinically important adverse experiences.

Accordingly, both levocetirizine and montelukast are pregnancy category B in the United States and are FDA approved in the United States down to six months of age for other disease processes. Moreover, both drugs have only once daily dosing, and no routine monitoring of blood work is necessary for most clinical situations. Further, both drugs exhibit minimal clinically relevant interactions with other medications. As described herein, administered orally, both levocetirizine and montelukast reach steady state levels within two days to rapidly produce a synergistic and complementary anti-inflammatory effect.

Administration of montelukast and a second generation antihistamine, fexofenadine, has a synergistic effect in the treatment of allergic rhinitis. Allergic rhinitis, also known as pollenosis or hay fever, is an allergic inflammation of the nasal airways which occurs when an allergen such as pollen or dust is inhaled by an individual with a genetically susceptible immune system (estimated at greater than 20 percent of the population). The allergen triggers antibody production, a serum specific immunoglobulin E (IgE), which in turn can bind to mast cells and basophils containing histamine. Upon re-exposure to the offending antigen, histamine is released causing the itching, swelling, and mucus production which are well known to seasonal allergy suffers. A combination of montelukast and fexofenadine reduced nasal congestion both subjectively, using patient diary and VAS evaluations, and objectively, using rhinomanometry and physical examination, with statistical significance compared to fexofenadine alone or fexofenadine with placebo.

However, the scientific literature does not clearly indicate whether the combination of an antihistamine plus a leukotriene offers an advantage over each alone for treatment in general. For example, in one chronic inflammatory disease state, chronic idiopathic urticaria, montelukast did not appear to offer an advantage over the second generation antihistamine desloratadine. See DiLorenzo G, et. al. Randomized placebo-controlled trial comparing desloratadine and montelukast in combined therapy for chronic idiopathic urticaria. J Allergy Clin Immunol 2004; 114-:619-25. Further, the FDA in April 2008 did approve the combination of loratadine, also a second generation antihistamine, and montelukast for the treatment of allergic rhinitis and asthma, finding no benefit from a combined pill.

Here; we describe the unexpected synergistic effects of combining levocetirizine and montelukast. Not wishing to be bound by a particular theory, a detailed examination of the pharmacokinetics of levocetirizine at the cell level illuminates the unique inflammatory properties that extend beyond the IgE mediated release of histamine. Levocetirizine exhibits a low volume of distribution (0.4L/kg), prolonged dissolution time from the H1 receptor in an acidic ph, enhanced receptor affinity as a pure isomer of cetirizine, and the highest receptor occupancy at 24 hours of any currently available antihistamine. Such parameters impart an inflammatory effect by down regulating IL-4, IL-6, IL-8 as well as cellular adhesion molecules. The later are a homogeneous group of inducible immunoglobulins, integrins and selectins involved in cell-to-cell adhesion, cellular recruitment, homing and healing. In addition levocetirizine has been shown in vivo to decrease ICAM-1, IL-6, IL-8, TLR3 expression and NF-kappa B activation resulting in decreased human rhinovirus titers by log-2. Many rhinovirus serotypes share the same cellular receptor identifying ICAM-1 as the portal of entry into the cell. Levocetirizine inhibits rhinovirus-induced ICAM-1 and cytokine expression and viral replication in airway epithelial cells. One log reduction in viral shedding results in a significant clinical benefit in HRV-infected (human rhinovirus) patients.

An unmet clinical need arose in 2009 with the H1N1 pandemic. The primary drug of choice for influenza, oseltamivir, did not appear to reduce influenza related lower respiratory tract complications. For neuraminidase inhibitors, there was a shortening of the illness by only one half to one day, which indicated that neuraminidase inhibitors do not prevent infection or stop nasal viral excretion, and therefore may be a suboptimal means of interrupting viral spread in a pandemic. Moreover, during this time frame, California reported alarming data on the severity of H1N1 influenza in pregnant and postpartum women, i.e., from April 23 through August 11, 2009 22% of pregnant or postpartum women required intensive care for the treatment of H1N1 and 8% died. Clinically it was demonstrated that the combination of levocetirizine plus montelukast (the latter added to protect the lower airway; both of which were Pregnancy Category B), could be safely and effectively used to ameliorate/shorten the course of influenza.

Not wishing to be bound by a particular theory, the steroid model suggests that levocetirizine acts in a non-lgE-mediated capacity at the level of NF-kB (See Figure 1) whereas montelukast acts at the CysLT1 receptor to inhibit the physiologic actions of LTD4. Both molecules are known to reduce the quantity of eosinophils or their migration to site of inflammation. Montelukast, in addition, also decreases the recruitment of dendritic cells and T cells.

The actions of levocetirizine plus montelukast surpass the individual physiologic mechanisms of each, well beyond the treatment of allergic rhinitis and asthma. At least in part, it is the anti-viral and anti-inflammatory properties of levocetirizine vis-a-vis nuclear factor kB; the inhibition of the actions of LTD4 by montelukast, underscored by ability of both levocetirizine and montelukast to inhibit the eosinophil quantity/migration, which impart synergy. This synergy is reflected by significantly improved clinical outcomes in a myriad of acute and chronic inflammatory disease states.

Disclosed herein are methods of treating inflammation of the entire respiratory tree, including in part, the nose and paranasal sinuses known as rhinosinusitis with montelukast and levocetirizine. Rhinosinusitis considered on a timeline may be acute, with a duration of less than six weeks (usually 4-6 weeks), subacute, having a duration of six to twelve weeks, or chronic, having a duration of greater than or equal to twelve weeks. Acute rhinosinusitis may be precipitated by multiple factors not limited to chemical irritation, trauma, allergic rhinitis or an earlier upper respiratory tract infection, which may be bacterial, viral, or, less commonly, fungal in origin. The most common causative agents of acute sinusitis of bacterial origin are Streptococcus pneumoniae, Haemophilus influenzae, Moraxella catarrhal's, Staphylococcus aureus, other streptococci species, anaerobic bacteria, and, less commonly, gram negative bacteria. Bacterial sinusitis tends to be more persistent than viral rhinosinusitis, i.e., the common cold, which typically lasts for 7 to 10 days.

Disclosed herein is the treatment of acute rhinosinusitis caused by a viral or bacterial infection with montelukast and levocetirizine According to the present disclosure, montelukast and levocetirizine are taken prophylactically to prevent a viral respiratory tract infection from escalating to an acute, often opportunistic, secondary bacterial sinusitis, bronchitis and/or pneumoniaAccording to the present disclosure, montelukast and levocetirizine are administered immediately, one hour. 6 hours, 12 hours, 1 day, 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, and/or 30 days after exposure to the pathogens (virus, bacteria, fungi; etc.). The present disclosure relates to the treatment of patients with clinical manifestations of influenza with montelukast and levocetirizine. According to the present disclosure, montelukast and levocetirizine treatment reduces the duration of influenza. According to the present disclosure, montelukast and levocetirizine treatment reduces the severity of influenza symptoms. Disclosed herein is the treatment of patients with clinical manifestations of the common cold with montelukast and levocetirizine. According to the present disclosure, montelukast and levocetirizine treatment reduces the duration of the cold. According to the present disclosure, montelukast and levocetirizine treatment reduces the severity of cold symptoms.

Chronic rhinosinusitis is an inflammatory condition/disease of the nose and paranasal sinuses lasting for greater than or equal to twelve weeks. Symptoms include in part, any combination of nasal congestion, facial pain, headache, coughing, an increase in asthma symptoms, malaise, discharge, feeling of facial tightness, dizziness, and/or aching teeth. Rhinosinusitis in general can be categorized into four categories: (1) acute bacterial rhinosinusitis (ABRS), (2) chronic rhinosinusitis without nasal polyposis (CRSsNP), (3) chronic sinusitis with nasal polyposis (CRSwNP), and (4) allergic fungal rhinosinusitis (AFRS). See Meltzer, EO. Rhinosinusitis: Developing guidance for clinical trials. J Allergy Clin Immunol 2006 November; S20. Nasal polyposis is a subgroup of chronic rhinosinusitis in which the inflammation of the nose is associated with two or more of the following signs and symptoms: nasal obstruction or congestion, nasal discharge, hyposmia or anosmia, facial pain or feeling of pressure, endoscopic evidence of polyps or mucopurulent discharge from middle meatus with or without edema or mucosal obstruction of the meatus and CT images which show mucosal changes of osteomeatal complex or paranasal sinuses. See Fokkens W, et. al. EAACI position paper on rhinosinusitis and nasal polyps executive summary. Allergy, 2005;60, 583-601., Fokkens, W, et. al. European Position Paper on Rhinosinusitis and Nasal Polyps group (2007) European position paper on rhinosinusitis and nasal polyps. Rhinology 2007;20,1-136. Conventional treatment for chronic rhinosinusitis often involves functional endoscopic sinus surgery, antibiotics, systemic and topical steroids, and to a much lesser extent an antihistamine or leukotriene modulator. The use of antihistamines in patients with only polyps has not been extensively studied. See Casale M, et. al. Nasal Polyposis: From Pathogenesis to Treatment, an Update. Inflammation & Allergy - Drug Targets 2011, 10, 158-163. Mometasone furoate monohydrate, a topical nasal steroid spray, is the only FDA approved medication in the United States for the treatment of nasal polyposis. The recommended dose is two squirts each nostril twice a day.

Disclosed herein is the treatment of chronic rhinosinusitis with montelukast and levocetirizine. Disclosed herein is the treatment of nasal polyposis with montelukast and levocetirizine. According to the present disclosure, montelukast and levocetirizine treatment reduces the size and/or number of polyps. Disclosed herein is the treatment of chronic rhinosinusitis with montelukast and levocetirizine in the absence of steroids, antibiotics or surgical treatment. According to the present disclosure, montelukast and levocetirizine are administered in conjunction with antibiotics and/or steroids and/or surgical treatment as deemed clinically applicable. The chronic rhinosinusitis treatment protocol with or without other treatment modalities is as follows:

**TABLE IV**

| TREATMENT PROTOCOL FOR CHRONIC RHINOSINUSITIS | |
|---|---|
| Levocetirizine - US | |
| Adults: | 5 mg / day |
| Children: 6-11 years of age: | 2.5 mg /day |
| Children: 6 months to 5 years | 1.25 mg / day |

| Montelukast - US | |
|---|---|
| Adults: | 10 mg orally /day |
| Children 6-14 years of age: | 5 mg orally/day |
| Children 6 months -5 years of age: | 4 mg orally / day |

Patients may be seen at least quarterly in the office with endoscopic review of the nose/paranasal sinuses when clinically appropriate. A pretreatment and follow-up CT scan of the perinasal sinuses at 6 months to one year post initiation of therapy may be performed to provide objective data on which to tailor existing medical therapy.

Disclosed herein is a method of treating rhinitis with montelukast and levocetirizine. Rhinitis, inflammation of the nasal passages, is commonly caused by a viral or bacterial infection, including the common cold, the latter of which is caused primarily by Rhinoviruses and Coronaviruses. See Eccles R. Understanding the Symptoms of the Common Cold and Influenza. Lancet Infectious Diseases 2005; 5(11): 718-725. Rhinitis is categorized as: (i) infective rhinitis; (ii) nonallergic rhinitis; and (iii) allergic rhinitis. Disclosed herein is a method of treating infective rhinitis with montelukast and levocetirizine. Disclosed herein is a method of treating nonallergic rhinitis with montelukast and levocetirizine. Disclosed herein is a method of treating allergic rhinitis with montelukast and levocetirizine.

Disclosed herein is the treatment of chronic rhinosinusitis with montelukast and levocetirizine. Disclosed herein is the treatment of chronic rhinosinusitis with montelukast and levocetirizine in the absence of steroid or antibiotic treatment. According to the present disclosure, montelukast and levocetirizine are administered in conjunction with antibiotics and/or steroids.

Disclosed herein is a method of treating non-lgE-based inflammation with montelukast and levocetirizine.

Disclosed herein is a method of treating combined IgE and non-lgE-mediated inflammation with montelukast and levocetirizine.

The following Table V shows the existing country guidelines for dosages in the treatment of allergic disorders.

**TABLE V**

| GUIDELINES FOR DOSAGES IN THE TREATMENT OF ALLERGIC DISORDERS | |
|---|---|
| Levocetirizine - US | |
| Adults: | 5 mg / day |
| Children: 6-11 years of age: | 2.5 mg /day |
| Children: 6 months to 5 years | 1.25 mg / day |

| Montelukast - US | |
|---|---|
| Adults: | 10 mg orally /day |
| Children 6-14 years of age: | 5 mg orally/day |
| Children 6 months -5 years of age: | 4 mg orally / day |

Disclosed herein is the use of a combination of levocetirizine and montelukast to treat a bacterial infection. Examples of bacterial infections that may be treated by a combination of levocetirizine and montelukast include, but are not limited to, acute bacterial rhinosinusitis (ABRS). According to the present disclosure, levocetirizine and montelukast may be administered with an antibiotic as determined by local presentation.

Disclosed herein is the use of a combination of levocetirizine and montelukast to treat otitis media with effusion and associated ear disorders such as chronic mastoiditis and eustachian tube dysfunction (the auditory tube leading from the back of the nose to the middle ear). According to the present disclosure, levocetirizine and montelukast may be administered with antibiotics to treat for example, acute otitis media with purulent middle ear effusion. In some embodiments, levocetirizine and montelukast may be administered without antibiotics to treat chronic middle ear effusion, for example, chronic otitis media. According to the present disclosure, levocetirizine and montelukast may be administered with other treatment modalities such as, but not limited to, steroids and/or antiviral agents.

Disclosed herein is the use of a combination of levocetirizine and montelukast to treat allergic fungal rhinosinusitis (AFRS). According to the present disclosure, levocetirizine and montelukast may be administered with other treatment modalities such as, but not limited to, steroids and/or an antifungal agent.

Intravenous therapy of levocetirizine and montelukast, the latter currently under investigation in the United States, would enhance the individual and combined clinical response presently seen with the administration of oral medication. The IV montelukast plasma concentration area under the curve profile, 7 mg, is comparable to the approved 10 mg oral montelukast tablet. The former has been shown in acute asthmatics to significantly improve FEV1 (forced expiratory volume at one sec) at 10 minutes when compared with placebo.

Accordingly, the dosing for acute inflammation could be daily as delineated above individually in the same setting, as a dual-layer tablet(s), and/or as a blister pack containing both medications for a 10 day course of therapy. For a moderate to severe clinical presentation, the levocetirizine component can be given at time zero (5 mg), 12 hours (5mg) and 24 hours (5 mg), during the first 24 hour day, in order to achieve a steady state level of the molecule in less than 40 hours. Levocetirizine human dosing safety studies have been performed at up to 30 mg/day. Sedation is the principal side effect experienced at higher doses. Independent research has shown that levocetirizine alone can be dosed at 20 mg /day to treat severe cases of idiopathic urticaria.

The application for the combination of levocetirizine and montelukast includes, but is not limited to treating, ameliorating, or preventing the following symptoms. For Influenza, the combination can be useful to shorten the course of seasonal flu and prevent or minimize the development of lower respiratory tract infections / complications, and/or to establish an improved, safe, world-wide protocol for influenza prior to the next pandemic, e.g., H5N1 with its associated 50% mortality rate. For upper respiratory tract infections, not limited to rhinovirus, the combination can be useful to limit the infection itself, and/or to prevent or reduce the potential development of secondary sinusitis, bronchitis and pneumonia. The combination can be useful for treatment of Ebstein-Barr Virus, particularly, but not limited to those patients with respiratory involvement.

For acute asthma in conjunction with existing protocols, not limited to exacerbations caused by rhinovirus (-50% of cases), the combination can be useful to shorten the course of the event, reduce hospitalizations and death. The combination can be useful for pre-treatment of patients allergic to one or more classes of antibiotics requiring antimicrobial therapy. These patients are at risk, 4-10x over the general population, of developing a subsequent ALE (allergic-like event). For patients with moderate to severe life-threatening disease requiring dual / triple antibiotics, the combination can be useful to reduce the probability of developing a side-effect(s) from the primary treatment medications. The combination can be useful during and following radiation therapy to ameliorate the inflammatory response. The combination can be useful for patients requiring steroids for the treatment of inflammation who are otherwise at increased risk for the development of steroid induced complications. Examples include but are not limited to the following: i) A severe insulin dependent diabetic with an infection such as facial paralysis, and ii) Patient with latent Tuberculosis. For patients on antiviral medication for acute disease, the combination can be used to prevent complications related to the medication(s) as well as complications associated with the disease process itself The combination can be used to treat serum sickness, with or without steroids. For pre-treatment of patients on immunotherapy, the combination can be used to prevent or ameliorate the risk of a systemic reaction. Examples of high risk patients with the potential to develop a life-threatening, systemic event include but are not limited to severe asthmatics, those patients with a concurrent respiratory tract infection, and those patients with a prior history of a systemic reaction. For pre and intra-treatment of those patients on chemotherapy, the combination can be used to ameliorate side effects associated with the administration of chemotherapeutic drug(s). For patients exhibiting a transfusion reaction, the combination can be used to limit the side effects/life threatening event during the initial reaction and in preparation for any requisite subsequent transfusion.

As will be readily apparent to one skilled in the art, the useful in vivo dosage of levocetirizine and montelukast to be administered and the particular mode of administration will vary depending upon the age, weight, medical condition of the patient, the severity of the condition to be treated, the route of administration, the renal and hepatic function of the patient, and mammalian species treated, the particular compounds employed, and the specific use for which these compounds are employed. The determination of effective dosage levels, that is the dosage levels necessary to achieve the desired result, can be accomplished by one skilled in the art using routine pharmacological methods. Typically, human clinical applications of products are commenced at lower dosage levels, with dosage level being increased until the desired effect is achieved. Advantageously, compounds of the present embodiments may be administered, for example, in a single daily dose, or the total daily dosage may be administered in divided doses of two, three or four times daily.

**TABLE VI**

| TREATMENT PROTOCOL FOR ACUTE INFLAMMATION NOT LIMITED TO INFLUENZA AND THE COMMON COLD | |
|---|---|
| Levocetirizine - US | |
| Adults: | 5 mg / day |
| Children: 6-11 years of age: | 2.5 mg /day |
| Children: 6 months to 5 years | 1.25 mg / day |

| Montelukast - US | |
|---|---|
| Adults: | 10 mg orally /day |
| Children 6-14 years of age: | 5 mg orally/day |
| Children 6 months -5 years of age: | 4 mg orally / day |

Depending upon the severity of the acute process, the doses in Table VI can be modified. For example, the age appropriate dose for levocetirizine may be given at time zero (at presentation) with an additional age appropriate dose at 12 hours. In order to protect the lower airway, particularly in the face of bronchitis/pneumonia, a dose of montelukast may be given at time zero (at presentation) with an additional age appropriate dose of montelukast at 12 hours. In this fashion the steady state level of the two drugs would approach 24 hours. Montelukast, like levocetirizine, is considered a very safe molecule. Montelukast has been administered at doses up to 200 mg/ day (20x the standard adult daily dose) to adult patients for 22 weeks and in short-term studies, up to 900 mg/day (90x the standard adult daily dose) to patients for approximately one week without clinically important adverse events. Dosing duration may parallel the generally accepted protocols for their respective disease states. For example, conventional therapy for an acute infectious disease process is typically administered for 5-14 days. A course of combined levocetirizine once daily plus montelukast once daily may be given for the same duration. For the treatment of chronic inflammatory disease states, an age appropriate once daily dosing of each medication may also be administered.

### Anaphylaxis

Several embodiments relate to the use of a combination of levocetirizine and montelukast for the treatment of anaphylaxis.

Anaphylaxis is defined as an acute, life-threatening systemic reaction with varied mechanisms, clinical presentations, and severity that results in the sudden release of mediators from mast cells and basophils. As used herein, the term "anaphylaxis" includes the following:

(1) Acute systemic reactions involving IgE-dependent mechanisms; (2) Acute systemic reactions involving other immunologic mechanisms (formerly called anaphylactoid reactions); (3) Acute systemic reactions that occur independently of any immunologic mechanism due to direct release of histamine and other mediators from mast cells and basophils, e.g., after exercise or exposure to cold or ultraviolet or ingestion of opioids, etc.; (4) Biphasic anaphylaxis, which is defined as a recurrence of symptoms that develops following the apparent resolution of the initial anaphylactic episode with no additional exposure to the trigger; and (5) Protracted anaphylaxis, which is defined as an anaphylactic reaction that last for hours days or even weeks in extreme cases.

Acute systemic reactions involving other immunologic mechanisms (formerly called anaphylactoid reactions) and acute systemic reactions that occur independently of any immunologic mechanism can exhibit identical clinical patterns to IgE-mediated anaphylaxis. Thus, as systemic events, they are treated the same.

Biphasic reactions have been reported to develop in up to 23% of anaphylactic episodes in adults and up to 11% of episodes and children; they typically occur within 8 to 10 hours after resolution of the initial symptoms although recurrences up to 72 hours later have been reported.

Disclosed herein is the combination of levocetirizine and montelukast for the treatment of IgE-mediated, non-lgE-mediated (other immunologic mechanisms or independently of any immunologic mechanism due to direct release of histamine and other mediators from mast cells), and/or combined non-lgE-mediated and IgE-mediated inflammation.

Food is the most common outpatient cause and accounts for 30% of fatal cases of anaphylaxis. Cutaneous signs and symptoms occur in 85-95% of cases followed by respiratory symptoms, i.e., shortness of breath and wheeze (45-50%), upper airway angioedema (50-60%) and rhinitis (15-20%). Table VII lists signs and symptoms of anaphylaxis; the data is a complication of 1865 patients and is adapted from Philip Lieberman, MD, et. al., The Diagnosis and Management of Anaphylaxis Practice Parameter: 2010 Update. J Allergy Clin Immunol; 126 (3): 480e1-42.

**TABLE VII**

| **SIGNS AND SYMPTOMS OF ANAPHYLAXIS** | |
|---|---|
| | Approximate Percentages |
| Cutaneous | |
| Urticaria and angioedema | 85-90 |
| Flushing | 45-55 |
| Pruritus without rash | 2-5 |
| Respiratory | |
| Dyspnea, wheeze | 45-50 |
| Upper airway angioedema | 50-60 |
| Rhinitis | 15-20 |
| Dizziness, syncope, hypotension | 30-35 |
| Abdominal | |
| Nausea, vomiting, diarrhea, cramping pain | 25-30 |
| Miscellaneous | |
| Headache | 5-8 |
| Substernal pain | 4-6 |
| Seizure | 1-2 |

The diagnoses of anaphylaxis is highly likely when any one of the following three criteria is fulfilled:
1. Acute onset of an illness (minutes to hours) with involvement of the skin, mucosal tissue, or both (e.g., generalized hives, pruritus, or flushing, swollen lips-tongue-uvula) and at least one of the following: respiratory compromise (e.g., dyspnea, wheeze-bronchospasm, stridor, reduced peak expiratory flow in older children, and hypoxemia in adults); and reduced blood pressure or associated symptoms of end-organ dysfunction (e.g., hypotonia, collapse, syncope, incontinence);
2. wo or more of the following occur rapidly after exposure to a likely allergen for that patient (minutes to hours): (1) Involvement of the skin-mucosal tissue (e.g., generalized hive, itch-flush, swollen lip-tongue-uvula); (2) Respiratory compromise (e.g., dyspnea, wheeze, bronchospasm, stridor, reduced PEF in older children and adults, hypoxemia); (3) Reduced blood pressure or associated symptoms (e.g., hypotonia, collapse, syncope, incontinence); and (4) Persistence gastrointestinal symptoms (e.g., cramping abdominal pain, vomiting); and
3. Reduced blood pressure after known exposure to a known allergen for that patient (minutes to several hours). Infants and children will have a low systolic blood pressure (age specific) or greater than 30% decrease in systolic blood pressure. Low systolic blood pressure for children is defined as: less than 70 mm Hg from one month to one year; less than 70 mmHg + (2X age) from one to 10 years, and less than 90 mm Hg from 11-17 years of age. Adults will have a systolic blood pressure of less than 90 mm Hg or greater than 30% decrease from that person's baseline.

Epinephrine is considered to be a first line medication for anaphylaxis treatment (See Table VIII, adapted from Simons, FE et al., World Allergy, Organization (2010), "World Allergy Organization survey on global availability of essentials for the assessment and management of anaphylaxis by allergy-immunology specialists in health care settings," Annals of Allergy, Asthma & Immunology: official publication of the American College of Allergy, Asthma, & Immunology 104 (5): 405-12. Herein incorporated by reference in its entirety). However, the drug can also cause pallor, tremor, anxiety, palpitations, dizziness, and headache when administered at a recommended dose; moreover, serious adverse effects, such as ventricular arrhythmias, hypertensive crisis, pulmonary edema, may also occur after an overdose of epinephrine.

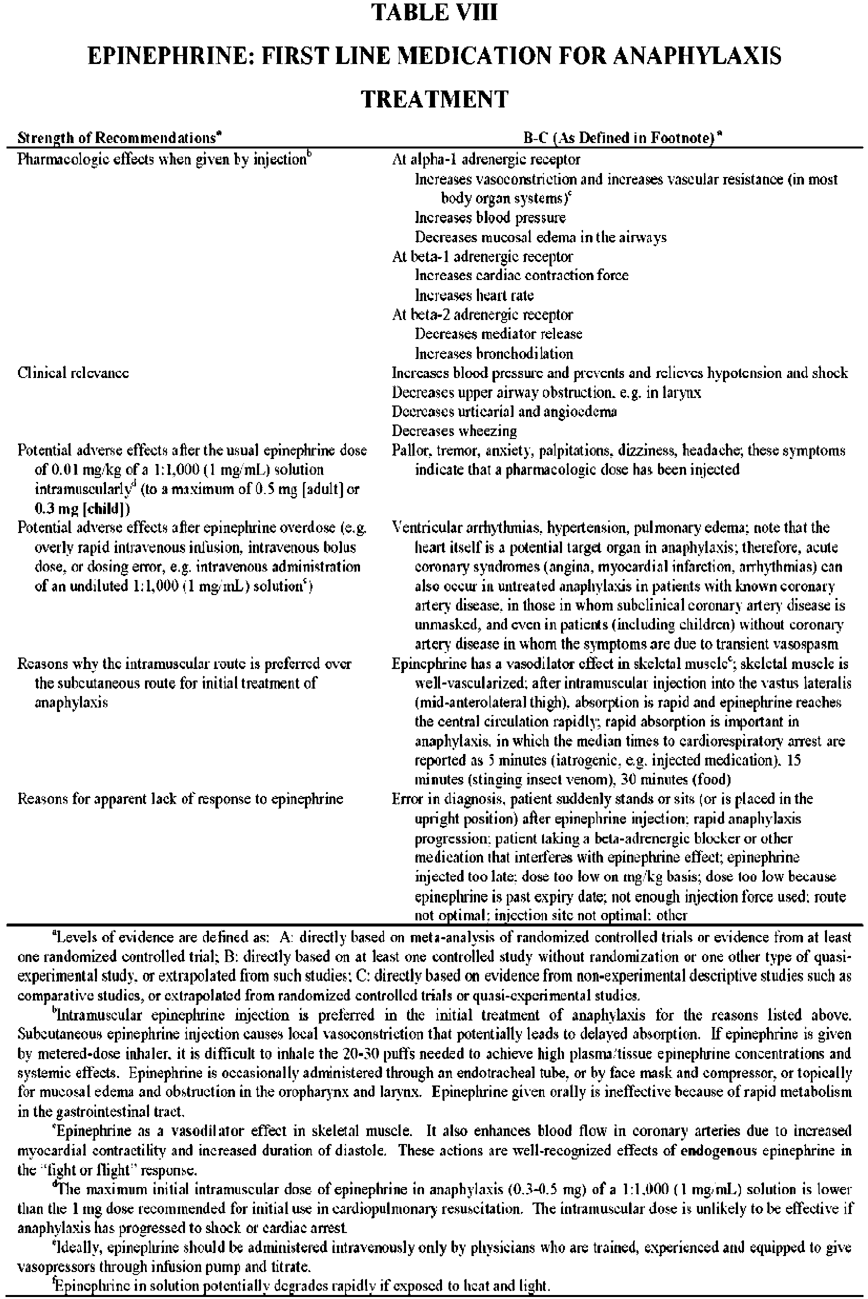

A detailed examination of the pharmacokinetics of levocetirizine at the cell level illuminates the unique anti-inflammatory properties that extend beyond the IgE mediated release of histamine. Most important are its low volume of distribution (0.4 L/kg; ideal drug < 0.6 L/kg), prolonged dissolution time from the H1 receptor in an acidic pH, enhanced receptor affinity as the pure isomer of cetirizine, fastest onset - 0.9 hour, fastest to steady state, approximately 40 hours, and the highest receptor occupancy at 24 hours (75%) of any currently available antihistamine. Such parameters impart an anti-inflammatory effect by down regulating IL-4, IL-6, IL-8 as well as cellular adhesion molecules. The latter are a homogeneous group of inducible immunoglobulins, integrins and selectins involved in cell-to-cell adhesion, cellular recruitment, homing and healing. IL-6 is particularly important as the signal protein for both fever and the acute phase response.

The cysteinyl leukotrienes (LTC4, LTD4, LDE4) are products of arachidonic acid metabolism. They promote accumulation and function of virtually all subgroups of leucocytes at the site of inflammation. Leukotrienes are released from various cells including mast cells and eosinophils. They bind to receptors in the human airway and on other pro-inflammatory cells including eosinophils and certain myeloid stem cells. The cysteinyl leukotrienes have been correlated with the pathophysiology of asthma and allergic rhinitis.

Leukotriene D4 (LTD4) a metabolite of leukotriene C4, is the most potent of the cysteinyl leukotrienes in contracting airway smooth muscle. It promotes the recruitment of eosinophils, dendritic cells (antigen presenting cells) and T cells, i.e. increases cell recruitment and activation and increases the Th2 inflammatory response. Montelukast specifically acts by binding with high affinity and selectivity to the CysLT1 receptor to inhibit the physiologic actions of LTD4.

Without being bound to a particularly theory, levocetirizine and montelukast work to block the H1 and leukotriene receptors, respectively. Thus, levocetirizine and montelukast quickly block the release of histamine to reduce systemic swelling and improved lung function by inhibiting the release of leukotrienes. Moreover, levocetirizine and montelukast, approximately 60 years newer than the prototype antihistamine, diphenhydramine, combined are scientifically more effective than its predecessor in stabilizing the airway and preventing cardiovascular collapse. Furthermore, the combination of levocetirizine and montelukast synergistically decrease eosinophil (the white blood cell considered the hallmark of inflammation) migration and quantity. Levocetirzine alone is known to block IL-6, the signaling protein responsible in part for the acute phase response and fever. However, the combination of levocetirizine and montelukast appear to work in separate sites of the steroid pathway (as shown in Figure 1) to augment and enhance effect of steroids by decreasing and/or blocking both the acute phase and late phase responses to the types of anaphylaxis described above (e.g. systemic reactions due to IgE, other immunologic mechanisms, or direct release of histamine and other mediators of inflammation). The combination of levocetirzine plus montelukast would additionally effectively treat or augment the treatment of biphasic and refractory (protracted) anaphylaxis.

Utilizing the combination of the leukotriene modulator montelukast plus the third generation antihistamine levocetirizine (hydroxyzine -cetirizine -levocetirizine) in anaphylaxis offers advantages over the current treatment paradigms. Both are pregnancy Category B, i.e., the safest for use in pregnancy and both are FDA approved for other uses down to age 6 months. Unlike other antihistamines, administration of levocetirizine and montelukast in combination exhibits synergistic effects and unexpectedly superior results in the treatment of anaphylaxis.

Moreover, combinations of levocetirizine and montelukast can be used safely in conjunction with many existing treatment protocols. For example, vasoconstrictors, such as epinephrine or dopamine, can be administered to a patient in combination with levocetirizine and montelukast. As an example, histamine H2 antagonists, including but not limited to ranitidine and cimetidine, may also be administered to a patient in combination with levocetirizine and montelukast. Other active agents, including but not limited to beta-2 agonists (a non-limiting example includes albuterol), glucocorticoids (non-limiting examples include hydrocortisone, methylprednisolone, prednisone, or prednisolone), and HI -antihisamines (non-limiting examples include chlorpheniramine, diphenhydramine, and cetirizine), may also be administered to a patient in combination with levocetirizine and montelukast (Table IX, adapted from Simons).

Moreover, a non-limiting example of how the present anaphylaxis treatment protocol may be refined includes: epinephrine, patient position (lying flat with the lower extremities elevated to preserve fluid in the circulation, prevent empty vena cava/empty ventricle syndrome, maintain the airway, and reduce the risk of aspiration), oxygen, intravenous saline, levocetirizine plus montelukast, an H2 receptor antagonist, and glucocorticoids. The combination of levocetirizine and montelukast may be administered orally, subcutaneously, intramuscularly or intravenously as adjunct therapy. In some embodiments, the combination of levocetirizine and montelukast may be administered intravenously (IV), for example, to quickly deliver the combination as an emergency combination, thereby aborting swelling and edema in minutes to complement epinephrine and oxygen far more effectively than diphenhydramine alone, which is now 70 years old.

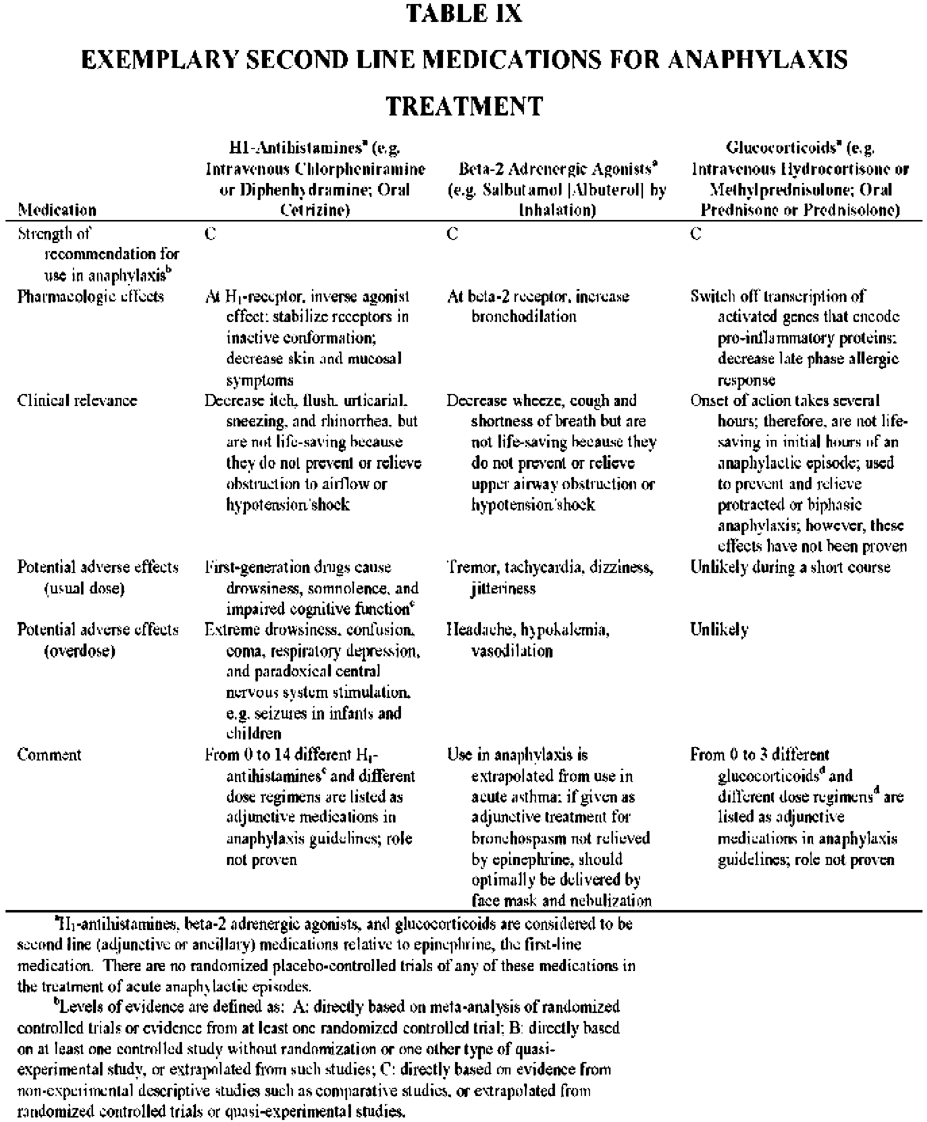

As will be readily apparent to one skilled in the art, the useful in vivo dosage of levocetirizine and montelukast to be administered and the particular mode of administration will vary depending upon the age, weight, medical condition of the patient, the severity of the condition to be treated, the route of administration, the renal and hepatic function of the patient, and mammalian species treated, the particular compounds employed, and the specific use for which these compounds are employed. The determination of effective dosage levels, that is the dosage levels necessary to achieve the desired result, can be accomplished by one skilled in the art using routine pharmacological methods. Typically, human clinical applications of products are commenced at lower dosage levels, with dosage level being increased until the desired effect is achieved. Advantageously, compounds of the present embodiments may be administered, for example, in a single daily dose, or the total daily dosage may be administered in divided doses of two, three or four times daily.

### Definitions

The term "effective amount" includes an amount effective, at dosages and for periods of time necessary, to achieve the desired result, e.g., sufficient to treat anaphylaxis and anaphylactic reactions in a patient or subject. An effective amount of levocetirizine and montelukast may vary according to factors such as the disease state, age, and weight of the subject, and the ability of levocetirizine and montelukast to elicit a desired response in the subject. Dosage regimens may be adjusted to provide the optimum therapeutic response. An effective amount is also one in which any toxic or detrimental effects (e.g., side effects) of levocetirizine and montelukast are outweighed by the therapeutically beneficial effects.

"Ameliorate," "amelioration," "improvement" or the like refers to, for example, a detectable improvement or a detectable change consistent with improvement that occurs in a subject or in at least a minority of subjects, e.g., in at least about 2%, 5%, 10%, 15%, 20%, 25%, 30%, 40%, 50%, 60%, 70%, 75%, 80%, 85%, 90%, 95%, 98%, 100% or in a range between any two of these values. Such improvement or change may be observed in treated subjects as compared to subjects not treated with levocetirizine and montelukast, where the untreated subjects have, or are subject to developing, the same or similar disease, condition, symptom or the like. Amelioration of a disease, condition, symptom or assay parameter may be determined subjectively or objectively, e.g., self-assessment by a subject(s), by a clinician's assessment or by conducting an appropriate assay or measurement, including, e.g., a quality of life assessment, a slowed progression of a disease(s) or condition(s), a reduced severity of a disease(s) or condition(s), or a suitable assay(s) for the level or activity(ies) of a biomolecule(s), cell(s), by detection of respiratory or inflammatory disorders in a subject, and/or by modalities such as, but not limited to photographs, video, digital imaging and pulmonary function tests. Amelioration may be transient, prolonged or permanent or it may be variable at relevant times during or after levocetirizine and montelukast are administered to a subject or is used in an assay or other method described herein or a cited reference, e.g., within timeframes described infra, or about 1 hour after the administration or use of levocetirizine and montelukast to about 28 days, or 1, 3, 6, 9 months or more after a subject(s) has received such treatment.

The "modulation" of, e.g., a symptom, level or biological activity of a molecule, or the like, refers, for example, to the symptom or activity, or the like that is detectably increased or decreased. Such increase or decrease may be observed in treated subjects as compared to subjects not treated with levocetirizine and montelukast, where the untreated subjects have, or are subject to developing, the same or similar disease, condition, symptom or the like. Such increases or decreases may be at least about 2%, 5%, 10%, 15%, 20%, 25%, 30%, 40%, 50%, 60%, 70%, 75%, 80%, 85%, 90%, 95%, 98%, 100%, 150%, 200%, 250%, 300%, 400%, 500%, 1000% or more or within any range between any two of these values. Modulation may be determined subjectively or objectively, e.g., by the subject's self-assessment, by a clinician's assessment or by conducting an appropriate assay or measurement, including, e.g., quality of life assessments, suitable assays for the level or activity of molecules, cells or cell migration within a subject and/or by modalities such as, but not limited to photographs, video, digital imaging and pulmonary function tests. Modulation may be transient, prolonged or permanent or it may be variable at relevant times during or after levocetirizine and montelukast are administered to a subject or is used in an assay or other method described herein or a cited reference, e.g., within times described infra, or about 1 hour after the administration or use of levocetirizine and montelukast to about 3, 6, 9 months or more after a subject(s) has received levocetirizine and montelukast.

As used herein, the terms "prevent," "preventing," and "prevention" refer to the prevention of the recurrence, onset, or development of anaphylaxis and anaphylactic reactions. Preventing includes protecting against the occurrence and severity of upper and/or lower respiratory tract infections.

As used herein, the term "prophylactically effective amount" refers to the amount of a therapy (e.g., a pharmaceutical composition comprising montelukast and levocetirizine) which is sufficient to result in the prevention of the development, recurrence, or onset of anaphylaxis and anaphylactic reactions; or to enhance or improve the prophylactic effect(s) of another therapy.

As used herein, "subject" includes organisms which are capable of suffering from anaphylaxis and anaphylactic reactions; or other disorder treatable by a combination of montelukast and levocetirizine or who could otherwise benefit from the administration of montelukast and levocetirizine as described herein, such as human and non-human animals. Preferred human animals include human subjects. The term "non-human animals" includes all vertebrates, e.g., mammals, e.g., rodents, e.g., mice, and non-mammals, such as non-human primates, e.g., sheep, dog, cow, chickens, amphibians, reptiles, etc.

The following Examples are presented for the purposes of illustration and should not be construed as limitations.

### EXAMPLES

### Example 1

Prophetic Case Study: 23-year-old female in her third month of pregnancy with a severe life-threatening allergic reaction to Augmentin® (amoxicillin/clavulanic acid).

### Chronology:

Patient is seen and evaluated by her doctor. The presentation included facial pain, pressure, low grade temperature of 99.5°F tympanic and a purulent nasal discharge. Following a diagnosis of acute bacterial sinusitis, a prescription for Augmentin® (amoxicillin/clavulanic acid) 875 mg, orally, twice daily forl 0 days is called in for patient convenience. The patient subsequently leaves the office and thereafter drives to the pharmacy. Upon receipt of the medication, she takes the first pill with water after being reassured by the pharmacist that the medication would not harm her baby. The patient had previously taken Augmentin® (amoxicillin/clavulanic acid) in the past without any side effects.

Approximately 20 minutes later, the patient experiences shortness of breath and a dry cough. The patient also notices a subtle swelling in the eyelids and face. A faint urticarial rash is developing on her right forearm. She immediately seeks medical attention at an emergency room. Medical professionals immediately notice the patient's pallor and shortness of breath and quickly initiate high flow oxygen via nasal prongs at 10 liters/minute and an IV, followed by a bolus of NaCl.

### Vital Signs on Admission:

| |
|---|
| B/P: 88/60 RR 24 |
| Heart rate 115 and regular T: 99.5° F |
| O2 saturation on room air: 92% |
| Weight: 165# / 75 kg |
| Height: 67" |

### Medications in the emergency room include the following:

| |
|---|
| Epinephrine (1:10,000 solution): 0.25 mg IV given x two, fifteen minutes apart |
| Levocetirizine 4 mg + montelukast 7 mg: IV to block the HI and leukotriene receptors, respectively. |
| Ranitidine: 50 mg IV to block the H2 receptors - decrease gastric secretions |

The patient survives the episode of anaphylaxis without complication. She delivers a healthy baby boy six months later.

### Example 2

Prophetic Case Study: 5-year-old male suffering from anaphylaxis from baklava containing tree nuts [based on an actual case].

A five year old boy weighing 44 pounds / 20 kg eats a piece of baklava brought over by friends on Christmas Day. He has eaten baklava and other tree-nut containing desserts in the past without incident. A few minutes later the child begins to develop a faint rash on his abdomen and stomach. Within twenty minutes he experiences swelling of the lips, bloating of his stomach, a diffuse and evolving maculopapular rash and dry cough. The boy is rushed to the local emergency room and immediately triaged to a gurney. In the interim, the rash has rapidly progressed to involve all four extremities, back, chest, and abdomen.

The emergency room physician immediately starts an IV in the left antecubital fossa and begins administration of a bolus of normal saline while the nurse secures nasal prongs delivering high flow oxygen at 8 liters/minute. The admission vital signs are: Temp 98°F tympanic in the right ear, Blood pressure: 68/40, respiratory rate 24, pulse 110, 88% 02 saturation on room air. IM epinephrine has already been administered in the right mid-anterolateral thigh. The dose is .01 mg/kg or 0.2mg for weight. Immediately thereafter the child is given levocetirizine 2 mg IV plus montelukast 4 mg IV. An additional dose of epinephrine is given 15 minute later to provide cardiovascular support followed by 4 mg of dexamethasone IV to block/reduce the late phase response to the systemic allergic reaction. The synergistic effect of levocetirizine plus montelukast will not only block the acute phase response but additionally complement the effect of dexamethasone; however, it takes at least four hour to witness a clinical response to dexamethasone.

The patient is stabilized and observed in the emergency room for five hours and then discharged home on a blister pack of levocetirizine 2.5 mg / montelukast 5 mg taken at night for seven days to prevent any biphasic reaction which could potentially follow the food associated anaphylaxis. His oxygen saturation which had dropped to 88% on presentation has risen to 98% on room air. At the time of discharge his rash has already improved.

Subsequent RAST (Radioallergoabsorbent testing, a blood test for allergy) one month later documents a RAST Class V/V to cashew, Class I/V to walnut and Class I/V to pecan. The RAST is scaled from I-V, with V being the highest. With a RAST Class V there is a 99% correlation between cause and effect.

Baklava is several countries is traditionally is made with one or more types of tree nuts and in the present case contained cashews, almonds, and pecan. The RAST test confirmed the diagnosis of the severe life-threatening systemic reaction to cashew.

The food associated anaphylaxis was successfully treated with a refined protocol containing levocetirizine plus montelukast which blocked both the acute and late phase response of the systemic allergic reaction.

Given the severe nature of the problem, Epi-Pens® (injectable epinephrine) and bilayer tablets containing levocetirizine 2.5 mg / montelukast 5 mg), are kept at school, at home and in glove boxes of both family vehicles for immediate use.

## Claims

1. A combination of levocetirizine and montelukast in an effective amount for use in the treatment of anaphylaxis or a symptom of anaphylaxis, or for use in reducing the duration of anaphylaxis in a patient in need thereof; wherein the combination is administered at the onset of symptoms.

2. The combination for use according to claim 1, wherein the combination is administered in a sequential manner.

3. The combination for use according to claim 1, wherein the combination is administered in a substantially simultaneous manner.

4. The combination for use according to claim 1, further comprising the administration of an additional active agent.

5. The combination for use according to claim 4, wherein the additional active agent is selected from the group consisting of a histamine H2 receptor antagonist, a beta2-agonist, oxygen, saline, a glucocorticoid, and a H1-anti-histamine.

6. The combination for use according to claim 5, wherein the histamine H2 antagonist is ranitidine.

7. The combination for use according to claim 5, wherein the histamine H2 antagonist is cimetidine.

8. The combination for use according to claim 1, further comprising the administration of a vasoactive agent.

9. The combination for use according to claim 8, wherein the vasoactive agent is epinephrine.

10. The combination for use according to claim 8, wherein the vasoactive agent is dopamine.

11. The combination for use according to claim 1, wherein the combination is administered to the patient by one or more of the routes consisting of enteral, intravenous, intraperitoneal, inhalation, intramuscular, subcutaneous and oral.

12. The combination for use according to claim 1, wherein the levocetirizine and montelukast are administered by the same route.

## Patentansprüche

1. Kombination von Levocetirizin und Montelukast in einer wirksamen Menge zur Verwendung bei der Behandlung von Anaphylaxie oder eines Anaphylaxiesymptoms, oder zur Verwendung bei der Verkürzung der Dauer von Anaphylaxie bei einem behandlungsbedürftigen Patienten; wobei die Kombination beim Beginn von Symptomen verabreicht wird.

2. Kombination zur Verwendung nach Anspruch 1, wobei die Kombination sequentiell verabreicht wird.

3. Kombination zur Verwendung nach Anspruch 1, wobei die Kombination im Wesentlichen gleichzeitig verabreicht wird.

4. Kombination zur Verwendung nach Anspruch 1, weiter umfassend die Verabreichung eines zusätzlichen aktiven Mittels.

5. Kombination zur Verwendung nach Anspruch 4, wobei das zusätzliche aktive Mittel aus der aus einem Histamin-H2-Rezeptorantagonisten, einem Beta2-Agonisten, Sauerstoff, einer Saline, einem Glukokortikoid und einem H1-Anti-Histamin bestehenden Gruppe ausgewählt ist.

6. Kombination zur Verwendung nach Anspruch 5, wobei der Histamin-H2-Antagonist Ranitidin ist.

7. Kombination zur Verwendung nach Anspruch 5, wobei der Histamin-H2-Antagonist Cimetidin ist.

8. Kombination zur Verwendung nach Anspruch 1, weiter umfassend die Verabreichung eines vasoaktiven Mittels.

9. Kombination zur Verwendung nach Anspruch 8, wobei das vasoaktive Mittel Epinephrin ist.

10. Kombination zur Verwendung nach Anspruch 8, wobei das vasoaktive Mittel Dopamin ist.

11. Kombination zur Verwendung nach Anspruch 1, wobei die Kombination über einen oder mehrere der Wege, bestehend aus enteraler, intravenöser, intraperitonealer, inhalativer, intramuskulärer, subkutaner und oraler Verabreichung, an den Patienten verabreicht wird.

12. Kombination zur Verwendung nach Anspruch 1, wobei das Levocetirizin und Montelukast über den gleichen Weg verabreicht werden.

## Revendications

1. Combinaison de levocetirizine et de montelukast d'une quantité efficace pour l'usage dans le traitement de l'anaphylaxie ou d'un symptôme de l'anaphylaxie, ou pour l'usage dans la réduction de la durée de l'anaphylaxie chez un patient nécessitant celui-ci; la combinaison étant administrée au début des symptômes.

2. Combinaison pour l'usage selon la revendication 1, dans laquelle la combinaison est administrée de manière séquentielle.

3. Combinaison pour l'usage selon la revendication 1, dans laquelle la combinaison est administrée d'une manière essentiellement simultanée.

4. Combinaison pour l'usage selon la revendication 1, comprenant en outre l'administration d'un agent actif supplémentaire.

5. Combinaison pour l'usage selon la revendication 4, dans laquelle l'agent actif supplémentaire est choisi dans le groupe constitué par un antagoniste du récepteur d'histamine H2, un agoniste bêta-2, l'oxygène, une solution saline, un glucocorticoïde et un antihistaminique H1.

6. Combinaison pour l'usage selon la revendication 5, dans laquelle l'antagoniste d'histamine H2 est ranitidine.

7. Combinaison pour l'usage selon la revendication 5, dans laquelle l'antagoniste d'histamine H2 est cimétidine.

8. Combinaison pour l'usage selon la revendication 1, comprenant en outre l'administration d'un agent vasoactif.

9. Combinaison pour l'usage selon la revendication 8, dans lequel l'agent vasoactif est l'épinéphrine.

10. Combinaison pour l'usage selon la revendication 8, dans lequel l'agent vasoactif est la dopamine.

11. Combinaison pour l'usage selon la revendication 1, dans laquelle la combinaison est administrée au patient par une ou plusieurs des voies consistant en entérale, intraveineuse, intrapéritonéale, par inhalation, par voie intramusculaire, sous-cutanée et orale.

12. Combinaison pour l'usage selon la revendication 1, dans laquelle la levocetirizine et le montelukast sont administrés par la même voie.
